(19) Europäisches Patentamt
European Patent Office
Office européen des brevets

(11) Veröffentlichungsnummer : **0 452 806 B1**

(12) # EUROPÄISCHE PATENTSCHRIFT

(45) Veröffentlichungstag der Patentschrift :
**19.01.94 Patentblatt 94/03**

(51) Int. Cl.$^5$ : **C07C 51/60,** C07C 53/42

(21) Anmeldenummer : **91105743.8**

(22) Anmeldetag : **11.04.91**

(54) **Verfahren zur Herstellung von Carbonsäurechloriden.**

(30) Priorität : **21.04.90 DE 4012781**

(43) Veröffentlichungstag der Anmeldung :
**23.10.91 Patentblatt 91/43**

(45) Bekanntmachung des Hinweises auf die Patenterteilung :
**19.01.94 Patentblatt 94/03**

(84) Benannte Vertragsstaaten :
**BE CH DE ES FR GB IT LI NL**

(56) Entgegenhaltungen :
**EP-A- 0 031 504**
**EP-A- 0 367 050**

(73) Patentinhaber : **BASF Aktiengesellschaft**
**Carl-Bosch-Strasse 38**
**D-67063 Ludwigshafen (DE)**

(72) Erfinder : **Kahl, Thomas-Michael, Dr.**
**Germersheimer Strasse 146**
**W-6725 Roemerberg (DE)**
Erfinder : **Henkelmann, Jochem, Dr.**
**Merianstrasse 5**
**W-6700 Ludwigshafen (DE)**
Erfinder : **Hupfer, Leopold, Dr.**
**Waltershoehe 3**
**W-6701 Friedelsheim (DE)**
Erfinder : **Franzischka, Wolfgang, Dr.**
**Flomersheimer Strasse 36**
**W-6710 Frankenthal (DE)**
Erfinder : **Schwarz, Wolfgang, Dr.**
**Frankenstrasse 24**
**W-6701 Otterstadt (DE)**

EP 0 452 806 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Carbonsäurechloride lassen sich gut durch Umsetzung der entsprechenden Carbonsäuren mit Phosgen herstellen. Die Reaktion bedarf der Katalyse. Als Katalysatoren kommen z.B. Carboxamide, vorzugsweise N-Alkylformamide zum Einsatz (DE-A-34 39 937).

Die Spanne der Alkylgröße reicht bei den N,N-Dialkylformamiden von Dimethylformamid bis zu Formamiden mit 30 C-Atomen (EP-A-0 050 779, DE-A-29 50 155, DE-A-19 31 074).

Durch die Wahl des Katalysatorsystems wird der Ablauf der Phosgenierung einer Carbonsäure zum Carbonsäurechlorid sowie die Aufarbeitung des Ansatzes entscheidend beeinflußt.

Alternativ zur Filtration von teerhaltigen Rohprodukten wäre in einigen Fällen auch eine destillative Aufarbeitung des katalysatorhaltigen Produktes denkbar. Die Destillation der entstandenen Säurechloride ist aber nicht nur ein energie- und zeitaufwendiger Vorgang, sondern birgt auch noch eine Reihe weiterer Nachteile.

Viele längerkettige Säurechloride lassen sich nicht ohne teilweise Zersetzung destillieren. Weiter ist bekannt, daß durch Zersetzung des im Destillationssumpf befindlichen Katalysators die destillierten Produkte verunreinigt werden können. Größere Mengen an Katalysatorrückstand stellen bei der Destillation ein Sicherheitsrisiko dar, weil in der Hitze die Gefahr einer spontanen Zersetzung besteht.

Bei der Produktaufarbeitung verunreinigter Ansätze vermindert sich sowohl durch Filtration als auch durch Destillation die Aktivität des Katalysators stark. In den meisten Fällen wird der eingesetzte Katalysator unbrauchbar, er kann also nicht wiederverwendet werden.

Sowohl die Destillation als auch die Filtration von katalysatorhaltigen Carbonsäurechloriden stellen also unvorteilhafte Aufarbeitungsmethoden dar. Wegen des durch die Aufarbeitung bedingten Katalysatorverlustes muß dessen Aufwandmenge so gering wie möglich ausfallen.

In der DE-A-29 50 155 wird als Katalysator Diisobutylformamid verwendet, das in jeder Phase der Umsetzung im Reaktionsansatz löslich ist. Soll auf eine abschließende Destillation des Säurechlorids verzichtet werden, muß aus Gründen der Produktreinheit der Anteil des löslichen Katalysators auf ein Minimum beschränkt werden. Auch bei diesem Katalysatorsystem ist eine Wiederverwendung des Katalysators ausgeschlossen, da er mit dem Produkt ausgetragen wird.

Es ist auch bekannt, daß die Umsetzungen mit Phosgen um so effektiver ablaufen, je größer der Katalysatoranteil ist. Umgekehrt bedingen geringe Katalysatormengen entweder eine schlechte Ausnutzung des eingegasten Phosgens oder lange Eingaszeiten.

In der DE-A-22 40 883 wird die Herstellung von Carbonsäurechloriden mit äquimolaren Mengen Carbonsäure und Katalysator beschrieben. Zur Abtrennung und Wiedergewinnung der großen Katalysatormenge ist allerdings die abschließende Zugabe des 3- bis 4-fachen Reaktionsvolumens an Benzol mit anschließender Destillation der benzolischen Produktlösung notwendig.

Den Einsatz großer Mengen Katalysator beschreibt auch JP-10 613/68 bei der Herstellung von Linolsäurechlorid mit 10 bis 50 mol-% Dimethylformamid, aber auch 1 bis 10 Äquivalenten Dimethylformamid, bezogen auf eingesetzte Linolsäure. Das entstandene Säurechlorid muß destilliert und zum Teil durch eine Behandlung mit Aktivkohle zusätzlich gereinigt werden. Die erneute Verwendung der großen Katalysatormengen ist nicht vorgesehen.

Bei der Synthese von Carbonsäuren mit Phosgen zu den entsprechenden Säurechloriden stellt sich bekanntermaßen das Problem, überschüssiges Phosgen aus dem rohen Säurechlorid zu entfernen.

Nach dem Stand der Technik kann phosgenhaltiges Carbonsäurechlorid durch mehrstündiges Strippen mit Stickstoff und/oder durch Anlegen eines leichten Vakuums von Phosgen befreit werden. Dieser Vorgang ist zeitraubend und verschlechtert die Raum-Zeit-Ausbeute des Verfahrens erheblich.

In der DE-A-29 50 155 wird das überschüssige Phosgen mit dem ersten Teil des destillierten Säurechlorids überdestilliert. Neben einer auch hier zu beobachtenden Verschlechterung der Raum-Zeit-Ausbeute erfordert diese Vorgehensweise zusätzlichen apparativen und analytischen Aufwand.

Aus der DE-A-22 40 883 ist eine Aufarbeitung bekannt, bei der vor der Destillation die verdünnte Reaktionslösung kurz mit Eiswasser gewaschen wird. Angesichts der Hydrolyseempfindlichkeit von Carbonsäurechloriden ist dieses Verfahren für den technischen Maßstab problematisch.

Auch bei dem aus JP 10613/68 bekannten Verfahren muß überschüssiges Phosgen durch eine destillative Aufarbeitung des rohen Säurechlorids entfernt werden.

Aus der DE-A-38 36 967 ist ein Verfahren zur Herstellung von höheren Carbonsäurechloriden bekannt, indem das N,N-Dialkylformamid zu 100 % mit Phosgen beladen wurde. Bei dieser Verfahrensweise entsteht ein nicht unerheblicher Phosgenverlust.

Der Erfindung lag nun die Aufgabe zugrunde, ein Verfahren zur Herstellung von Carbonsäurechloriden zu finden, das den vorgenannten Nachteilen abhilft.

Demgemäß wurde ein neues und verbessertes Verfahren zur Herstellung von Carbonsäurechloriden der

allgemeinen Formel I

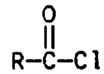

(I),

in der R für $C_1$- bis $C_{30}$-Alkyl, $C_2$- bis $C_{30}$-Alkenyl und $C_2$- bis $C_{30}$-Alkinyl steht, aus Carbonsäuren der allgemeinen Formel II

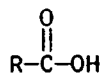

(II),

in der R die oben genannten Bedeutungen hat, und Phosgen, $COCl_2$ (III), in Gegenwart eines Katalysator-Addukts aus Phosgen und N,N-disubstituiertem Formamid der allgemeinen Formel IV

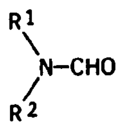

(IV),

in der $R^1$ und $R^2$ unabhängig voneinander $C_1$- bis $C_3$-Alkyl oder $R^1$ und $R^2$ gemeinsam eine $C_4$- oder $C_5$-Alkylenkette bedeuten, die gegebenenfalls durch ein Sauerstoffatom oder ein Stickstoffatom, das eine $C_1$- bis $C_3$-Alkylgruppe oder CHO trägt, unterbrochenen sein kann, wobei das Reaktionsprodukt I durch Phasentrennung erhalten wird, gefunden, dadurch gekennzeichnet, daß man das Formamid (IV) zu 20 bis 70 mol-% mit Phosgen belädt.

Die Carbonsäurechloride (I) sind nach folgender Methode erhältlich:

a) diskontinuierlich:

Zum vorgelegten Reaktionsgemisch, bestehend aus einer Carbonsäure (II) und dem Addukt aus Phosgen und N,N-Dialkylformamid der Formel (IV), wird eine der Carbonsäure (II) äquivalente Menge flüssiges oder gasförmiges Phosgen gegeben. Der Zeitbedarf für die Zufuhr des Phosgens kann dabei auf wenige Stunden begrenzt werden, wobei das Phosgen praktisch quantitativ ausgenutzt wird. Anschließend läßt man 1 bis 2 Stunden absitzen und trennt die Phasen.

b) bevorzugt kontinuierlich:

Als Reaktoren sind z.B. Rührkessel, Rührkesselkaskade oder eine im Gegenstrom betriebene Reaktionskolonne geeignet.

Bei Verwendung eines Rührkessels legt man die Carbonsäure (II) und das Addukt aus Phosgen und N,N-disubstituiertem Formamid der Formel (IV) vor und gibt flüssiges oder gasförmiges Phosgen zu. Nach Einleiten einer der Carbonsäure (II) äquivalenten Menge Phosgen beginnt man gleichzeitig Carbonsäure (II) und das Addukt aus Phosgen und N,N-disubstituiertem Formamid sowie eine im wesentlichen der zugefahrenen Carbonsäure (II) äquimolare Menge Phosgen zuzufahren.

Eine der den zugefahrenen Reaktanden entsprechende Menge des Reaktionsvolumens wird dem Kessel über eine Standhaltung entnommen und in ein Trenngefäß geleitet. Aus dem Trenngefäß kann das Wertprodukt (I) als Oberphase kontinuierlich entnommen und das Addukt aus Phosgen und N,N-disubstituiertem Formamid als Unterphase kontinuierlich in den Reaktor zurückgeführt werden. Um den Beladungsgrad des Katalysators während des kontinuierlichen Prozesses möglichst konstant zu halten, ist es aber sinnvoll, das durch die Reaktionsabgase mit-gerissene Phosgen durch zusätzlich zugeführtes Phosgen auszugleichen.

Die Temperaturen für die diskontinuierliche und die bevorzugte kontinuierliche Umsetzung der Carbonsäuren (II) mit dem Addukt aus Phosgen (III) und N,N-disubstituiertem Formamid (IV) liegen zwischen 20 und 100°C, vorzugsweise zwischen 30 bis 80°C, besonders bevorzugt zwischen 40 bis 70°C.

Bei dieser Umsetzung verwendet man 5 bis 200 mol-%, vorzugsweise 10 bis 100 mol-%, besonders 10 bis 30 mol-% des Addukts aus Phosgen und N,N-disubstituiertem Formamid, bezogen auf die eingesetzte Carbonsäure.

Der Beladungsgrad des Katalysators liegt zwischen 20 und 70 mol-%, vorzugsweise 30 bis 60 mol-%, besonders 40 bis 50 mol-%.

Der Beladungsgrad des Katalysators kann kontinuierlich durch Messung der Dichte, der Viskosität, der Leitfähigkeit oder des Chlorid-Gehalts der im Trenngefäß anfallenden schwereren Phase bestimmt werden.

Die Phasentrennung erfolgt bei Temperaturen von -15 bis 40°C, vorzugsweise -10 bis 30°C, besonders - 5 bis 20°C.

Die eingesetzte Phosgenmenge (III) ist im wesentlichen äquimolar zur Carbonsäure (II).

Da das Reaktionsabgas neben Kohlendioxid und Chlorwasserstoff nur unwesentliche Mengen Phosgen enthält (1,0 bis 1,5 %) ist eine Kondensation des Phosgens nicht erforderlich. Die Abgase können direkt der Abgaswäsche zugeführt werden. Zur Kühlung des Abgases sind deshalb nur solche Temperaturen notwendig, die das gebildete Wertprodukt möglichst vollständig kondensieren.

Dem Reaktionsgemisch kann, zur besseren Phasentrennung oder zur Umsetzung fester Säuren, ein unter diesen Reaktionsbedingungen inertes Lösungsmittel zugesetzt werden, wie z.B. gesättigte aliphatische Kohlenwasserstoffe, Ether, Acetonitril, Benzol, Toluol oder Cyclohexan.

Das Carbonsäurechlorid fällt in hoher Ausbeute und in hoher Reinheit an. Es kann häufig ohne weitere Reinigung weiterverwendet werden. In einigen Fällen ist zur Erlangung hochreiner Produkte eine einfache Destillation erforderlich. Die Carbonsäurechloride fallen nach der Umsetzung phosgenfrei an und alle Maßnahmen zur Entphosgenierung des rohen Säurechlorids entfallen.

Das erfindungsgemäße Verfahren zur Herstellung von Säurechloriden aus aliphatischen Carbonsäuren eignet sich vor allem für Monocarbonsäuren, d.h. zur Herstellung von Verbindungen mit der allgemeinen Formel RCOX, wobei R eine aliphatische Kohlenwasserstoffgruppe darstellt und X für Chlor steht. Die aliphatische Gruppe kann geradkettig oder verzweigt, gesättigt, olefinisch oder acetylenisch ungesättigt sein. Besonders bevorzugt sind aliphatische Carbonsäuren mit 1 bis 30, insbesondere 1 bis 20 Kohlenstoffatomen.

Als N,N-disubstituierte Formamide eignen sich Dimethyl-, Ethyl-methyl-, Methyl-n-propyl-, Methyl-isopropyl-, Diethyl-, Ethyl-n-propyl, Ethyl-isopropyl-, Di-n-propyl-, n-Propyl-iso-propyl- und Di-iso-propylformamid, bevorzugt sind Dimethyl- und Diethylformamid, besonders bevorzugt ist Dimethylformamid.

Beispiele

Beispiele 1 bis 8 (kontinuierliche Phosgenierung)

In einem 350 ml Reaktor (HR) wird bei 40 bis 70°C 1,0 mol/h einer Carbonsäure, 0,2 Mol/h des Addukts aus Phosgen und N,N-disubstituiertem Formamid mit einem Beladungsgrad von 50 bis 60 % sowie 1,0 Mol/h Phosgen zugefahren. Das auf 20°C abgekühlte Abgas (Flußwasser) wird direkt über einen Wäscher geleitet, um nicht umgesetztes Phosgen zu hydrolysieren.

Nach der Nachreaktion in einem zweiten Reaktor (NR) bei 40 bis 65°C werden Säurechlorid- und Katalysatorphase bei Raumtemperatur in einem Phasentrenngefäß getrennt und anschließend die Zusammensetzung des Säurechlorids sowie der Beladungsgrad des Katalysators bestimmt. Alle Austräge waren phosgenfrei.

Die Ergebnisse sind in Tabelle 1 zusammengestellt.

Tabelle 1

| Bsp. Nr. | Säure | Katalysator | Temp. [°C] HR | Temp. [°C] NR | Säurechlorid ber. rein [%] | Säurechlorid Gehalt [%] | Beladungsgrad Kat. [mol-%] vorher | Beladungsgrad Kat. [mol-%] nachher | Phosgenverlust [%] |
|---|---|---|---|---|---|---|---|---|---|
| 1 | Propion-säure | N,N-Dimethyl-formamid | 40 | 40 | 73 | 96,4 | 60 | 43 | 3,4 |
| 2 | Pivalin-säure | N,N-Dimethyl-formamid | 50 | 50 | 94 | 98,0 | 60 | 50 | 2,0 |
| 3 | 2-Ethyl-hexansäure | N,N-Dimethyl-formamid | 50 | 50 | 99 | 99,5 | 50 | 44 | 1,2 |
| 4 | Stearin-säure | N,N-Dimethyl-formamid | 70 | 65 | 99 | 99,5 | 50 | 38 | 2,4 |
| 5 | Pivalin-säure | N,N-Diethyl-formamid | 60 | 60 | 90 | 95,1 | 60 | 49 | 2,2 |
| 6 | 2-Ethyl-hexansäure | N,N-Diethyl-formamid | 60 | 60 | 98 | 99,5 | 50 | 42 | 1,6 |
| 7 | 2-Ethyl-hexansäure | N-Formyl-piperidin | 50 | 50 | 98 | 99,0 | 50 | 41 | 1,8 |
| 8 | Isobutter-säure[a)] | N,N-Dimethyl-formamid | 45 | 50 | 97 | 98,5 | 50 | 45 | 1,0 |

a) Kühlung des Abgas mit -15°C-Sole

Vergleichsbeispiele 9 bis 14 (diskontinuierliche Phosgenierung nach DE-A-38 36 987)

In einem 1 l-Reaktor werden 2 Mol einer Carbonsäure und 0,4 Mol eines N,N-disubstituierten Formamids vorgelegt und bei 50 bis 70°C in einem Zeitraum von 2 h 238 g (2,4 Mol) Phosgen eingegast. Das auf 20°C abgekühlte Abgas (Flußwasser) wird direkt über einen Wäscher geleitet, in dem unumgesetztes Phosgen hydrolysiert wird.

Nach Ende der Phosgenzugabe wird 0,5 h nachgerührt, der Ansatz auf Raumtemperatur abgekühlt und zur Phasentrennung in einen Scheidetrichter überführt. Nach 0,5 h bei 25°C wird der Austrag in Säurechlorid und Katalysatorphase getrennt. Alle Austräge fallen phosgenfrei an.

Die Ergebnisse sind in Tabelle 2 zusammengestellt.

Tabelle 2

| Bsp. Nr. | Säure | Katalysator | Temp. [°C] | Säurechlorid ber. rein [%] | Säurechlorid Gehalt [%] | Beladungsgrad Katalysator [mol-%] | Phosgenverlust [%] |
|---|---|---|---|---|---|---|---|
| 9 | 2-Ethylhexan-säure | N,N-Dimethyl-formamid | 50 | 95 | 99,3 | 54 | 7,7 |
| 10 | Pivalin-säure | N,N-Diethyl-formamid | 60 | 91 | 95,1 | 49 | 8,5 |
| 11 | 2-Ethyl-hexansäure | N,N-Diethyl-formamid | 60 | 97 | 99,1 | 60 | 6,9 |
| 12 | Stearin-säure | N,N-Diethyl-formamid | 70 | 96 | 97,0 | 49 | 8,5 |
| 13 | Pivalin-säure | N-Formyl-piperidin | 60 | 92 | 98,1 | 58 | 7,1 |
| 14 | 2-Ethyl-hexansäure | N-Formyl-formamid | 60-70 | 98 | 99,2 | 72 | 4,7 |

Beispiele 15 bis 21 (Katalysator-Rückführung)

In einem 350 ml Reaktor (HR) wird bei 40 bis 70°C 1 bis 2 mol/h einer Carbonsäure mit 20 mol-% des Addukts aus Phosgen und N,N-disubstituierten Formamids mit einem Beladungsgrad von 50 bis 60 % und sowie Phosgen zugeführt, daß neben der Umsetzung zum Säurechlorid auch der Phosgenverlust durch das Abgas ausgeglichen wird. Die Abgase werden durch Solekühlung auf -20°C abgekühlt.

Nach der Nachreaktion in einem zweiten Reaktor (NR) bei 40 bis 65°C werden die Säurechlorid- und Katalysatorphase bei Raumtemperatur in einem Phasentrenngefäß getrennt. Der Katalysator wird nach Ergänzung des mit der Säurechloridlösung ausgetragenen N,N-disubstituierten Formamids zurückgeführt. Die Austräge sind jederzeit phosgenfrei.

Die Ergebnisse sind in Tabelle 3 zusammengestellt.

Tabelle 3

| Bsp. Nr. | Säure | Mol/h | Kat. | Belad. Kat. [mol-%] | Temp. [°C] HR | Temp. [°C] NR | Phosgen [Mol/h] | Rückführung | Wertprodukt [Flächen-%] Säurechlorid | Wertprodukt [Flächen-%] Formamid | Phosgenverlust [%] |
|---|---|---|---|---|---|---|---|---|---|---|---|
| 15 | Propionsäure | 1,20 | DMF a) | 60 | 40 | 40 | 1,25 | 1 | 97,2 | 1,1 | 4,0 |
| | | 1,10 | | | | | 1,13 | 2 | 96,4 | 1,5 | 2,7 |
| 16 | iso-Buttersäure | 1,00 | DMF a) | 55 | 45 | 50 | 1,01 | 1 | 98,8 | 0,6 | 1,0 |
| | | 1,50 | | | | | 1,52 | 2 | 98,5 | 0,7 | 1,3 |
| | | 2,00 | | | | | 2,04 | 3 | 98,1 | 0,8 | 1,9 |
| | | 2,00 | | | 40 | 45 | 2,05 | 4 | 98,8 | 0,8 | 2,4 |
| | | 2,00 | | | 45 | 20 | 2,05 | 5 | 98,1 | 0,5 | 2,4 |
| 17 | 2-Ethylhexansäure | 1,30 | DMF a) | 50 | 50 | 50 | 1,32 | 1 | 99,4 | 0,1 | 1,5 |
| | | | | | | | | 2 | 99,5 | 0,2 | 1,5 |
| | | | | | | | | 3 | 99,7 | 0,1 | 1,5 |
| 18 | Pivalinsäure | 1,40 | DMF a) | 50 | 50 | 50 | 1,42 | 1 | 98,6 | 0,3 | 1,5 |
| | | | | | | | 1,43 | 2 | 99,1 | 0,6 | 2,1 |
| 19 | Stearinsäure | 0,80 | DMF a) | 45 | 70 | 60 | 0,82 | 1 | 99,5 | 0,1 | 2,4 |
| | | 1,00 | | | | | 1,03 | 2 | 99,4 | 0,1 | 2,9 |
| 20 | Pivalinsäure | 1,70 | DEF b) | 60 | 60 | 60 | 1,75 | 1 | 95,1 | 2,5 | 2,8 |
| | | | | | | | 1,75 | 2 | 94,5 | 3,2 | 2,8 |
| 21 | 2-Ethylhexansäure | 1,30 | DEF b) | 50 | 60 | 60 | 1,32 | 1 | 97,8 | 1,1 | 1,5 |
| | | | | | | | 1,32 | 2 | 97,4 | 1,3 | 1,5 |
| | | | | | | | 1,33 | 3 | 97,5 | 1,3 | 2,2 |

a) DMF ≙ N,N-Dimethylformamid
b) DEF ≙ N,N-Diethylformamid

## Patentansprüche

1. Verfahren zur Herstellung von Carbonsäurechloriden der allgemeinen Formel I

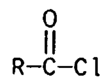 (I),

in der R für $C_1$- bis $C_{30}$-Alkyl, $C_2$- bis $C_{30}$-Alkenyl und $C_2$- bis $C_{30}$-Alkinyl steht, aus Carbonsäuren der allgemeinen Formel II

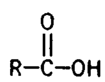 (II),

in der R die oben genannten Bedeutungen hat, und Phosgen, $COCl_2$ (III), in Gegenwart eines Katalysator-Addukts aus Phosgen und N,N-disubstituiertem Formamid der allgemeinen Formel IV

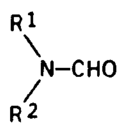 (IV),

in der $R^1$ und $R^2$ unabhängig voneinander $C_1$- bis $C_3$-Alkyl oder $R^1$ und $R^2$ gemeinsam eine $C_4$- oder $C_5$-Alkylenkette bedeuten, die gegebenenfalls durch ein Sauerstoffatom oder ein Stickstoffatom, das eine $C_1$- bis $C_3$-Alkylgruppe oder CHO trägt, unterbrochenen sein kann, wobei das Reaktionsprodukt I durch Phasentrennung erhalten wird, dadurch gekennzeichnet, daß man das Formamid (IV) zu 20 bis 70 mol-% mit Phosgen belädt.

2. Verfahren nach Anspruch 1, dadurch gekennzeichnet, daß man die Umsetzung kontinuierlich durchführt.

## Claims

1. A process for preparing carbonyl chlorides of the general formula I

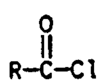 (I),

where R is $C_1$-$C_{30}$-alkyl, $C_2$-$C_{30}$-alkenyl or $C_2$-$C_{30}$-alkynyl, from carboxylic acids of the general formula II

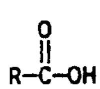 (II),

where R is as defined above, and phosgene, $COCl_2$ (III), in the presence of a catalyst adduct of phosgene and an N,N-disubstituted formamide of the general formula IV

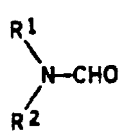 (IV),

where $R^1$ and $R^2$ are each independently of the other $C_1$-$C_3$-alkyl or together a $C_4$- or $C_5$-alkylene chain, which may be interrupted by an oxygen atom or a nitrogen atom which carries a $C_1$-$C_3$-alkyl group or CHO, the reaction product I being obtained by phase separation, which comprises loading the formamide (IV) with phosgene to an extent of from 20 to 70 mol%.

**2.** A process as claimed in claim 1, wherein the reaction is carried out continuously.

## Revendications

**1.** Procédé pour préparer des chlorures d'acides carboxyliques répondant à la formule générale I

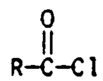

(I),

dans laquelle R est un radical alkyle en $C_1$-$C_{30}$, alcényle en $C_2$-$C_{30}$ ou alcynyle en $C_2$-$C_{30}$, à partir d'acides carboxyliques répondant à la formule générale II

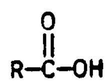

(II),

dans laquelle R a les significations ci-dessus, et de phosgène $COCl_2$ (III), en présence d'un catalyseur produit d'addition de phosgéne et d'un formamide N,N-disubstitué répondant à la formule générale IV

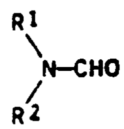

(IV),

dans laquelle $R^1$ et $R^2$, indépendamment l'un de l'autre, représentent chacun un radical alkyle en $C_1$-$C_3$, ou bien $R^1$ et $R^2$ forment ensemble un enchaînement alkylène en $C_4$- $C_5$, qui éventuellement peut être interrompu par un atome d'oxygène ou un atome d'azote portant un groupe alkyle en $C_1$-$C_3$ ou CHO, le produit de réaction I étant obtenu par séparation des phases, caractérisé en ce qu'on charge le formamide (IV) d'une quantité de phosgène comprise entre 20 et 70 % en moles.

**2.** Procédé selon la revendication 1, caractérisé en ce que la réaction est mise en oeuvre d'une manière continue.